(19) Europäisches Patentamt
European Patent Office
Office européen des brevets

(11) **EP 4 763 048 A1**

(12) **EUROPEAN PATENT APPLICATION**
published in accordance with Art. 153(4) EPC

(43) Date of publication:
**24.06.2026 Bulletin 2026/26**

(21) Application number: **24861963.7**

(22) Date of filing: **03.09.2024**

(51) International Patent Classification (IPC):
*A61B 3/10* (2006.01)    *A61B 3/13* (2006.01)
*A61B 34/20* (2016.01)    *G01B 9/02* (2022.01)

(86) International application number:
**PCT/CN2024/116613**

(87) International publication number:
**WO 2025/051120 (13.03.2025 Gazette 2025/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.09.2023   CN 202311146993**

(71) Applicant: **Towardpi (Beijing) Medical Technology Ltd.**
**Beijing 102206 (CN)**

(72) Inventors:
• **YANG, Zhi**
  **Beijing 102206 (CN)**
• **XU, Guojun**
  **Beijing 102206 (CN)**
• **SHEN, Shilin**
  **Beijing 102206 (CN)**
• **FU, Mingzhu**
  **Beijing 102206 (CN)**
• **WANG, Xiao**
  **Beijing 102206 (CN)**

(74) Representative: **Murgitroyd & Company**
**165-169 Scotland Street**
**Glasgow G5 8PL (GB)**

(54) **INTRAOPERATIVE OCT NAVIGATION METHOD AND SYSTEM FOR OPHTHALMIC OPERATING MICROSCOPE, AND OPHTHALMIC OPERATING MICROSCOPE**

(57)    The present disclosure relates to the technical field of ophthalmic surgical microscopes and discloses an intraoperative OCT navigation method and system for an ophthalmic surgical microscope, and the ophthalmic surgical microscope. The method includes: obtaining, by a scanning galvanometer, 3D OCT raw scanning data of a patient's ocular region; performing signal processing on the obtained 3D OCT raw scanning data to obtain a first A-scan signal of an OCT image; and displaying a 3D OCT image based on the obtained first A-scan signal of the OCT image. The system includes a scanning galvanometer, a signal processing device and a display device. The microscope includes the system.

Fig. 1

## Description

### TECHNICAL FIELD

The present disclosure relates to the technical field of ophthalmic surgical microscopes, and specifically relates to an intraoperative OCT navigation method and system for an ophthalmic surgical microscope, and the ophthalmic surgical microscope.

### BACKGROUND

Optical Coherence Tomography (OCT) has been widely used for diagnosis of ocular diseases, which is of great significance for detection and treatment of ocular region diseases.

Nowadays, the intraoperative OCT navigation system for an ophthalmic surgical microscope is typically a 2D OCT navigation system that has the problem of being unable to display, or missing, image details.

### SUMMARY

The present disclosure provides an intraoperative OCT navigation method and system for an ophthalmic surgical microscope, and the ophthalmic surgical microscope, to enable a display of a 3D OCT image.

In accordance with a first aspect of the present disclosure, there is provided an intraoperative navigation OCT navigation method for an ophthalmic surgical microscope, comprising:

> obtaining, by a scanning galvanometer, 3D OCT raw scanning data of a patient's ocular region;

> performing signal processing on the obtained 3D OCT raw scanning data to obtain a first A-scan signal of an OCT image; and

> displaying a 3D OCT image based on the obtained first A-scan signal of the OCT image.

In accordance with another aspect of the present disclosure, there is provided an intraoperative OCT navigation system for an ophthalmic surgical microscope, comprising:

> a scanning galvanometer configured to obtain 3D OCT raw scanning data of a patient's ocular region;

> a signal processing device configured to perform signal processing on the obtained 3D OCT raw scanning data to obtain a first A-scan signal of an OCT image; and

> a display device configured to display a 3D OCT image based on the first A-scan signal of the OCT image.

In accordance with a further aspect of the present disclosure, there is provided an ophthalmic surgical microscope, comprising the intraoperative OCT navigation system for the ophthalmic surgical microscope according to any of the embodiments of the present disclosure.

In the technical solution according to the embodiments of the present disclosure, during an ocular surgery based on an ophthalmic surgical microscope, a scanning galvanometer can be used to obtain 3D OCT raw scanning data by scanning a ocular region; signal processing is performed on the 3D OCT raw scanning data to obtain a first A-scan signal of an OCT image; and based on the obtained first A-scan signal of the OCT image, a 3D OCT image is displayed, to enable intraoperative 3D OCT navigation. As compared with 2D OCT navigation, the intraoperative 3D OCT navigation can improve the display effect of the intraoperative OCT image of the ophthalmic surgical microscope.

It would be appreciated that this Summary is not intended to identify key features or essential features of the present disclosure, nor is it intended to be used to limit the scope of the present disclosure. Other features of the present disclosure would be made more apparent through the following description.

### BRIEF DESCRIPTION OF THE DRAWINGS

In order to make clearer the technical solution according to the embodiments of the present disclosure, brief introduction of the drawings required in the embodiments will be provided below. Apparently, the drawings depicted below only relate to some embodiments of the present disclosure, and the ordinary skill in the art could derive other drawings on the basis of those drawings, without doing creative work.

> Fig. 1 is a flowchart of an intraoperative OCT navigation method for an ophthalmic surgical microscope provided by an embodiment of the present disclosure;

> Fig. 2A is a schematic diagram of grid points in a single-line scanning mode provided by an embodiment of the present disclosure;

> Fig. 2B is a schematic diagram of grid points in a cross scanning mode provided by an embodiment of the present disclosure;

> Fig. 2C is a schematic diagram of grid points in a grid scanning mode provided by an embodiment of the present disclosure;

> Fig. 2D is a schematic diagram of grid points in a spiral scanning mode provided by an embodiment of the present disclosure;

Fig. 3 is a schematic flowchart of processing OCT raw scanning data provided by an embodiment of the present disclosure;

Fig. 4 is a rendered-enhanced image of intraoperative OCT navigation fundus volume data provided by an embodiment of the present disclosure;

Fig. 5 is a flowchart of another intraoperative OCT navigation method for an ophthalmic surgical microscope provided by an embodiment of the present disclosure;

Fig. 6 is a schematic diagram of triangulation of spiral scanning coordinate points provided by an embodiment of the present disclosure;

Fig. 7 is a flowchart of a further intraoperative OCT navigation method for an ophthalmic surgical microscope provided by an embodiment of the present disclosure;

Fig. 8 is a schematic diagram of a structure of an intraoperative OCT navigation system for an ophthalmic surgical microscope provided by an embodiment of the present disclosure; and

Fig. 9 is a schematic diagram of a structure of an ophthalmic surgical microscope provided by an embodiment of the present disclosure.

## DETAILED DESCRIPTION OF EMBODIMENTS

In order to enable those skilled in the art to better understand the solution of the present disclosure, reference below will be made to the drawings according to the embodiments of the present disclosure to provide clear and complete description of the technical solution according to the present disclosure. Obviously, the embodiments described herein are only a part of the embodiments of the present disclosure, not all of them. On the basis of the embodiments of the present disclosure, the ordinary skill in the art could derive all the other embodiments, without doing creative work, which should all fall into the scope of protection of the present disclosure.

It is worth noting that the terms "first", "second", and the like, as used in the description, claims and drawings of the present disclosure, are only intended for distinguishing similar objects, but not necessarily require a specific order or sequence. It would be appreciated that these terms could be used interchangeably, if appropriate, indicating that the embodiments of the present disclosure can be carried out in other orders than those depicted or described herein. In addition, the terms "include", "comprise", or any other variant thereof are intended to cover a non-exclusive inclusion. For example, a process, method, product, or device including a series of steps or units are not necessarily limited to those steps or units listed explicitly, or further cover other steps or units not listed clearly or inherent in the process, method, product, or device.

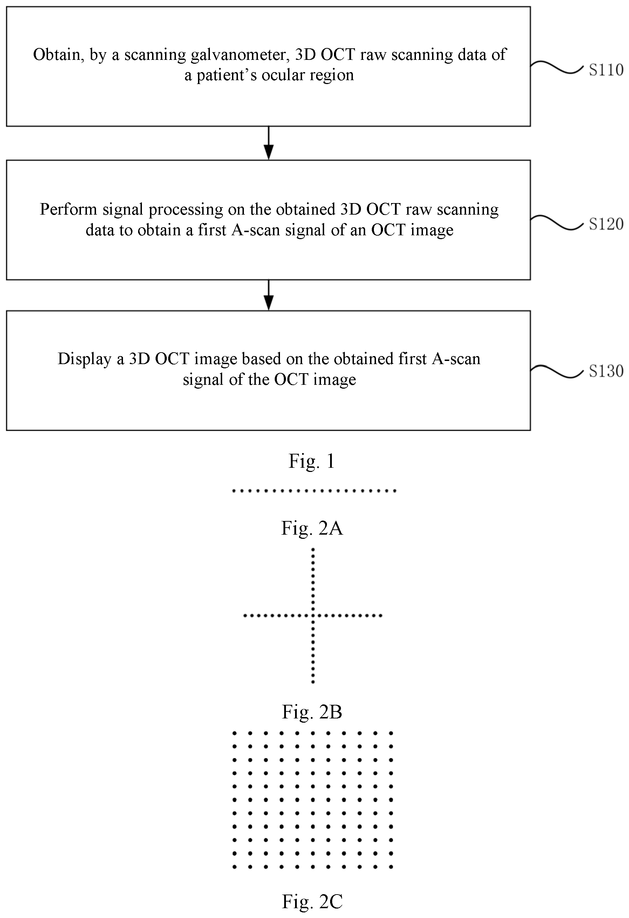

Fig. 1 is a flowchart of an intraoperative OCT navigation method for an ophthalmic surgical microscope provided by an embodiment of the present disclosure. The embodiment can be applied to a scene of anterior segment imaging and/or posterior segment imaging. The method can be performed by an intraoperative OCT navigation system for an ophthalmic surgical microscope, which can be implemented in the form of hardware and/or software, and configured in an ophthalmic surgical microscope.

In the embodiment of the present disclosure, the ophthalmic surgical microscope includes: a scanning galvanometer, a signal processing device and a display device, where the scanning galvanometer is used for scanning a patient's ocular region in real time and obtaining a scanning signal. In the scanning process of the scanning galvanometer, light emitted from the light source is split into two beams, namely: one beam to be emitted to the ocular region, which is referred to as signal arm, and the other beam to be emitted to a reference reflecting mirror, which is referred to as reference arm. Further, the scanning signal can be transmitted to a signal acquisition card through an optical path structure of the surgical microscope, and then converted by the signal acquisition card into 3D OCT raw scanning data that can be digitally processed. The signal acquisition card is electrically connected to the signal processing device, such that the signal processing device can receive the 3D OCT raw scanning data and perform signal processing on the obtained 3D OCT raw scanning data to obtain a first A-scan signal of an OCT image; the signal processing device is electrically connected to the display device such that the display device can display a 3D OCT image based on the obtained first A-scan signal of the OCT image.

As shown in Fig. 1, the method includes:

Step S110: obtaining, by a scanning galvanometer, 3D OCT raw scanning data of a patient's ocular region.

In the embodiment, the scanning galvanometer of the ophthalmic surgical microscope can be used to scan the patient's ocular region in real time to obtain corresponding 3D OCT data which is the 3D OCT raw scanning data.

By way of example, in the 3D OCT scanning mode, the 3D OCT raw scanning data may be 3D OCT data collected from the patient's left or right ocular region, where the collected 3D OCT raw scanning data may include a reference clock signal (clock) on the reference arm and a sample signal (signal) on the signal arm.

In the embodiments of the present disclosure, the ophthalmic surgical microscope may have a 3D OCT scanning mode only. Alternatively, in other embodiments of the present disclosure, the ophthalmic surgical microscope may have both a 3D OCT scanning mode and a 2D OCT scanning mode, thus making the OCT navigation display more diversified. In an optional embodiment, when the ophthalmic surgical microscope has both the 3D OCT scanning mode and the 2D OCT scanning mode, in response to that the ophthalmic surgical microscope starts the 3D OCT scanning mode or is switched from the 2D OCT scanning mode to the 3D OCT scanning mode, the 3D OCT raw scanning data can be obtained using the scanning galvanometer, to implement 3D scanning for the ocular region and enable 3D OCT navigation.

In the embodiments of the present disclosure, the display device can send a scanning waveform signal corresponding to the 3D OCT scanning mode to the scanning galvanometer, and upon receiving the scanning waveform signal corresponding to the 3D OCT scanning mode, the scanning galvanometer performs a scanning operation on the patient's ocular region to obtain 3D OCT raw scanning data. Wherein, the display device may be a personal computer or a server with a display function, or the like, which is not specifically limited herein.

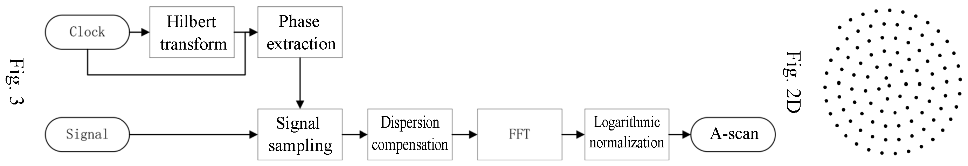

By way of example, the 2D OCT scanning mode may include, but is not limited to, a single-line scanning mode, a cross scanning mode, or the like, which is not specifically limited herein. Fig. 2A is a schematic diagram of grid points in a single-line scanning mode provided by an embodiment of the present disclosure; and Fig. 2B is a schematic diagram of grid points in a cross scanning mode provided by an embodiment of the present disclosure. It is worth noting that the 2D OCT raw scanning data collected in the single-line or cross scanning mode can enable an axial image display. By way of example, the 3D OCT scanning mode may include, but is not limited to, a grid scanning mode, a spiral scanning mode, or the like, which is not specifically limited herein. Fig. 2C is a schematic diagram of grid points in a grid scanning mode provided by an embodiment of the present disclosure; and Fig. 2D is a schematic diagram of grid points in a spiral scanning mode provided by an embodiment of the present disclosure. It is worth noting that the 3D OCT raw scanning data is collected in the mesh or spiral scanning mode. The 3D OCT scanning data obtained in the grid scanning mode is 3D volume data that can be displayed. Although not being displayable 3D volume data, the 3D OCT scanning data obtained in the spiral scanning mode can be restored as displayable 3D volume data through grid restoration, to enable a display of a 3D OCT image.

In a further optional embodiment, in response to that the ophthalmic surgical microscope starts the 2D OCT scanning mode or is switched from the 3D OCT scanning mode to the 2D OCT scanning mode, 2D OCT raw scanning data can be obtained through the scanning galvanometer, and then, signal processing can be performed on the obtained 2D OCT raw scanning data, to obtain a second A-scan signal of an OCT image; and the 2D OCT image can be displayed based on the obtained second A-scan signal of the OCT image.

In the embodiments of the present disclosure, the display device can send a scanning waveform signal corresponding to the 2D OCT scanning mode to the scanning galvanometer. Upon receiving the scanning waveform signal corresponding to the 2D OCT scanning mode, the scanning galvanometer performs a 2D scanning operation on the patient's ocular region, to obtain 2D OCT raw scanning data. 2D OCT raw scanning data includes a clock signal and a sample signal; then, phase information of the clock signal is used to perform digital signal processing on the sample signal, to obtain a second A-scan signal of an OCT image; further, the second A-scan signal of the OCT image is directly displayed visually to obtain a 2D OCT image, thus enabling intraoperative 2D OCT navigation.

In the embodiment, the ophthalmic surgical microscope may be provided with a 400K/100K frequency-variable light source. In the 3D OCT scanning mode, the sweeping rate of the 400K/100K frequency - variable light source of the ophthalmic surgical microscope can be changed to 400K, to scan the patient's ocular region at a high speed, and thus improve the real-time display rate of the 3D OCT image. In addition/alternatively, in the 2D OCT scanning mode, the sweeping rate of the 400K/100K frequency - variable light source of the ophthalmic surgical microscope can be changed to 100K, to improve the display effect of the 2D OCT image and increase the OCT scanning depth.

In a further embodiment, in the 3D OCT scanning mode, the sweeping rate of the frequency - variable light source of the ophthalmic surgical microscope can be changed to 500K or the like, and in the 2D OCT scanning mode, the sweeping rate of the variable-frequency light source of the ophthalmic surgical microscope can be changed to 150K or the like, which is not specifically limited herein.

Step S120: performing signal processing on the obtained 3D OCT raw scanning data to obtain a first A-scan signal of an OCT image.

In the embodiment, the 3D volume data obtained after the 3D OCT raw scanning data undergoes the signal processing is the first A-scan signal of the OCT image, which may include depth scanning information. It is worth noting that any A-scan signal has a scanning grid point corresponding thereto, i.e., the first A-scan signal of the OCT image may consist of a plurality of A-scan signals.

By way of example, Fig. 3 is a schematic flowchart of processing OCT raw scanning data provided by an embodiment of the present disclosure. In the embodiment of the present disclosure, the step of performing signal processing on the 3D OCT raw scanning data may include, but is not limited to: performing Hilbert transform on the clock signal in the 3D OCT raw scanning data to obtain a clock signal having been subject to the Hilbert transform, then performing phase extraction on the clock signal having been subject to the Hilbert transform to obtain phase information corresponding to the clock signal, generating a time index corresponding to the clock signal based on the phase information corresponding to the clock signal, and subsequently interpolating the sample signal based on the time index corresponding to the clock signal to obtain interpolated sample signal, where the interpolation processing method may be linear interpolation, spline interpolation or the like, which is not limited herein; further, performing dispersion compensation, Fast Fourier Transform (FFT) and logarithmic normalization operations on the interpolated sample signal to obtain the first A-scan signal of the OCT image which may be an 8-bit 2048-point image sequence.

With the signal processing described above, the first A-scan signal of the OCT image can be obtained based on the clock signal and the sample signal in the 3D OCT raw scanning data. As compared with the manner of directly sending the 3D OCT raw scanning data to the display device to display the 3D OCT image, this manner of sending the first A-scan signal of the processed OCT image to the display device to display the 3D OCT image can reduce the amount of data transmitted per unit of time while improving stability and reliability of data transmission.

Step S130: displaying a 3D OCT image based on the obtained first A-scan signal of the OCT image.

In the embodiments of the present disclosure, the first A-scan signal of the OCT image can be rendered in real time using a set imaging algorithm to obtain a 3D OCT image.

In the embodiment, one or more of the following operations are performed on the first A-scan signal of the OCT image to obtain a 3D OCT image that can be displayed visually, including: performing adaptive sampling processing on the first A-scan signal of the OCT image; performing random jitter processing on the first A-scan signal of the OCT image; performing pseudo-color processing on the first A-scan signal of the OCT image; performing visual enhancement processing on the first A-scan signal.

In the embodiments of the present disclosure, adaptive sampling method is used for sampling during a volume ray casting process to enhance a contrast between a non-uniform area and a uniform area in the 3D OCT image. In order to eliminate a wood-grain artifact caused by undersampling, in the embodiment, a random small offset can be added to a light sampling position in the observation direction, according to the random jitter technology, thus improving the wood - grain artifact caused by a low sampling rate. Further, pseudo-color coloring can be performed on the first A-scan signal based on an intensity signal, and a Phong illumination model is used to perform visualization enhancement on the OCT image, to improve the 3D OCT image quality.

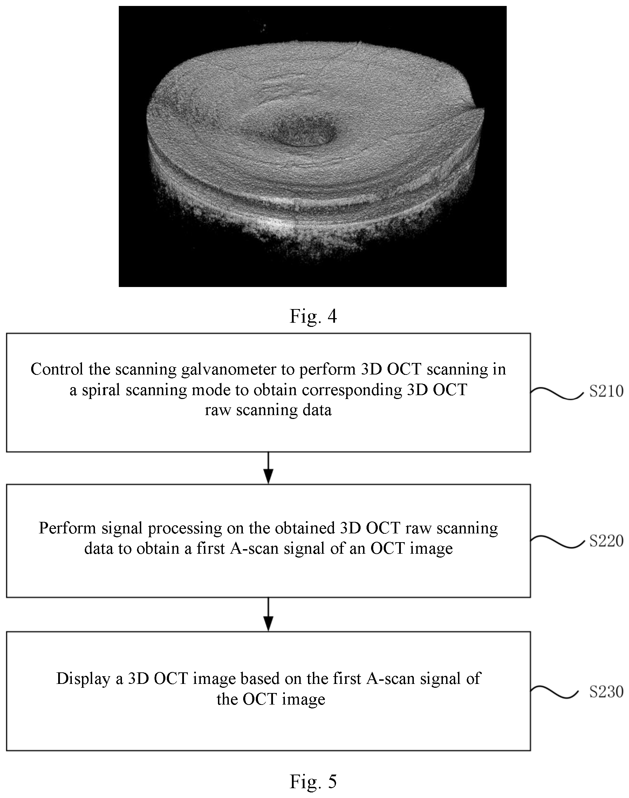

By way of example, Fig. 4 is a rendered-enhanced image of intraoperative OCT navigation fundus volume data provided by an embodiment of the present disclosure. In the embodiment of the present disclosure, Fig. 4 is a rendered-enhanced image of a posterior segment rendered with the set imaging algorithm. As seen from Fig. 4, the contrast of the posterior segment is significantly enhanced in the image while the existing problems of a blurred structure and a low edge clarity are improved effectively.

In some embodiments, in order to improve the rendering rate, a GPU can be used to render and display the first A-scan signal of the OCT image.

In the technical solution according to the embodiments of the present disclosure, the scanning galvanometer is used to obtain 3D OCT raw scanning data; signal processing is performed on the 3D OCT raw scanning data to obtain a first A-scan signal of an OCT image; based on the obtained first A-scan signal of the OCT image, a 3D OCT image is displayed, to enable intraoperative 3D OCT navigation. As compared with 2D OCT navigation, the intraoperative 3D OCT navigation can cause more details to be displayed, thus improving the display effect of the intraoperative OCT of the ophthalmic surgical microscope.

Fig. 5 is a flowchart of another intraoperative OCT navigation method for an ophthalmic surgical microscope provided by an embodiment of the present disclosure. The method according to the embodiment can be combined with respective optional solutions about the intraoperative OCT navigation methods for the ophthalmic surgical microscope provided in the embodiments described above.

As shown in Fig. 5, the method includes:
Step S210, controlling the scanning galvanometer to perform 3D OCT scanning in a spiral scanning mode, to obtain corresponding 3D OCT raw scanning data.

In a further optional embodiment, the scanning galvanometer can also be controlled to perform 3D OCT scanning in a grid scanning mode, to obtain corresponding 3D OCT raw scanning data.

As shown in Fig. 2C, in the grid scanning mode, the scanning time of the scanning galvanometer during the return from the right end to the left end is 25% of the scanning time from the left end to the right end. Since the scanning time is shortened, the 3D OCT scanning data collected by scanning during the return from the right end to the left end may be invalid data, resulting in occurrence of invalid data in each scanning cycle, which reduces the efficiency of obtaining 3D OCT scanning data. In addition, in the grid scanning mode, there is a higher performance requirement imposed on the x-scanning galvanometer of the scanning galvanometer, i.e., the x-scanning galvanometer should have a high scanning frequency, which, however, may cause a damage to the x-scanning galvanometer. In the spiral scanning mode, the collected 3D OCT scanning data is all valid data, thus improving effectiveness of scanning data collection. Moreover, the spiral scanning mode has a low requirement for the scanning frequency of the x-scanning galvanometer and the y-scanning galvanometer. Accordingly, in the mode, a scanning galvanometer with a lower performance can be used to obtain data with the same efficiency as in the grid scanning mode, and thus the scanning galvanometer is not easily damaged. Further, after the grid point data is restored, only the grid point data within a predetermined circular area can be displayed as actually required.

Step S220: performing signal processing on the obtained 3D OCT raw scanning data to obtain a first A-scan signal of an OCT image.

Step S230: displaying a 3D OCT image based on the obtained first A-scan signal of the OCT image.

As an optional embodiments, the step S230 may include: converting the obtained first A-type signal of the OCT image into corresponding grid point data, and then displaying a 3D OCT image in real time.

It is worth noting that, in the single-line scanning mode or the cross scanning mode, the obtained 2D A-scan signal can be displayed directly. However, in the spiral scanning mode according to the embodiment, due to the spiral scanning characteristics, it is required that the first A-type signal be converted into grid point data before the 3D OCT image is displayed in real time.

In an optional embodiment, nearest-interpolation processing can be performed on the first A-scan signal of the OCT image, i.e., for any grid point, the A-scan of the spiral scanning coordinate point with the smallest distance from the grid point is used as data of the grid point to implement conversion of the grid point data.

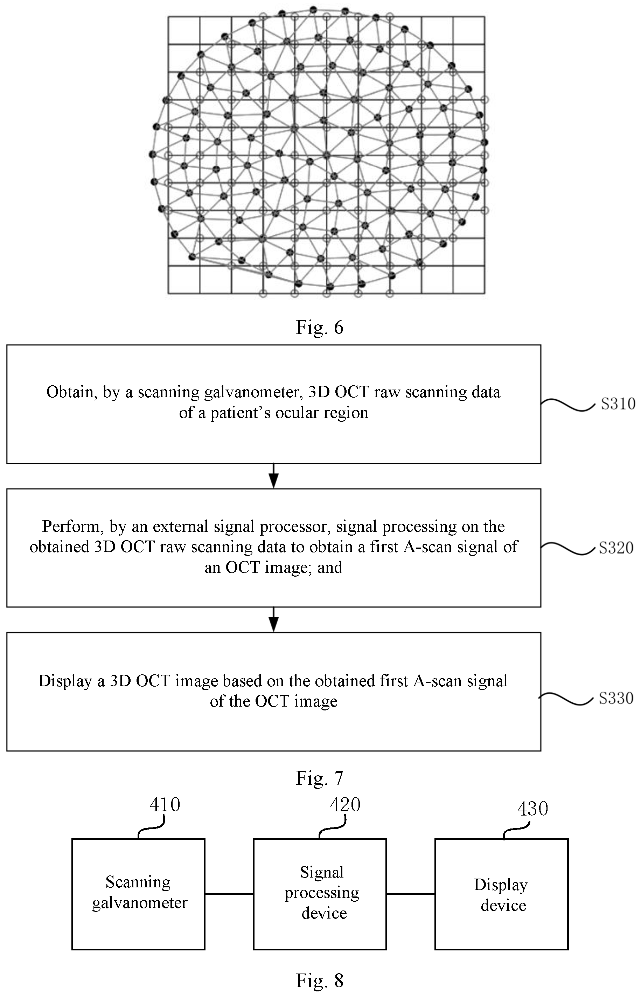

In a further optional embodiment, triangulation interpolation can be performed on the first A-scan signal of the OCT image to obtain grid point data corresponding to the first A-scan signal. By way of example, Fig. 6 is a schematic diagram of triangulation of spiral scanning coordinate points provided by an embodiment of the present disclosure. In Fig. 6, the solid dots represent spiral scanning coordinate points obtained through spiral scanning, and the hollow dots represent grid coordinate points to be reconstructed. In the embodiments, for a grid point i located in solid dots a, b and c (a, b, c and i are not shown in Fig. 6), assumed that the distances from the grid point i respectively to the solid dots a, b and c are $d_{ai}$, $d_{bi}$, and $d_{ci}$, the A-scan corresponding to the grid point i is:

$$I(i) = \frac{d_{ai}{}^2 * I(a) + d_{bi}{}^2 * I(b) + d_{ci}{}^2 * I(c)}{d_{ai}{}^2 + d_{bi}{}^2 + d_{ci}{}^2},$$

where I(a) is the A-scan of the solid dot a, I(b) is the A-scan of the solid dot b, and I(c) is the A-scan of the solid dot c. It is worth noting that, as compared with the nearest-neighbor interpolation, the triangulation interpolation is more accurate and brings about a better effect of restored grid point data.

By controlling the scanning galvanometer to perform 3D OCT scanning in the spiral scanning mode, the technical solution according to the embodiments of the present disclosure can effectively improve effectiveness of data collection, and has a low requirement for the scanning frequency of the scanning galvanometer, thus avoiding damage to the scanning galvanometer while improving the operation stability of the device.

Fig. 7 is a flowchart of a further intraoperative OCT navigation method for an ophthalmic surgical microscope provided by an embodiment of the present disclosure. The method according to the embodiment can be combined with respective optional solutions about the intraoperative OCT navigation methods for the ophthalmic surgical microscope provided in the embodiments described above.

As shown therein, the method includes:
Step S310: obtaining, by the scanning galvanometer, 3D OCT raw scanning data.

In the embodiment of the present disclosure, the scanning mode of the scanning galvanometer may include, but is not limited to, a grid scanning mode, a spiral scanning mode, and the like, which is not specifically limited herein.

Step S320: performing, by an external signal processor, signal processing on the obtained 3D OCT raw scanning data to obtain a first A-scan signal.

Wherein, the external signal processor refers to a signal processing device independent of the display device. For example, the signal processor may be a Digital Signal Processing (DSP) device, a Field Programmable Gate Array (FPGA), or the like, which is not specifically limited herein.

In the embodiment of the present disclosure, the external signal processor may be an FPGA integrated on a signal acquisition card.

By way of example, after the scanning galvanometer scans the patient's ocular region, the reflected light signal is transmitted to the signal acquisition card through the optical path structure of the surgical microscope, and the signal acquisition card then converts the light signal into an electrical signal; then, the electrical signal is converted into 3D OCT raw scanning data that can be digitally processed; subsequently, an onboard FPGA performs digital signal processing on the 3D OCT raw scanning data to obtain a first A-scan signal of an OCT image, and transmits the first A-scan signal of the OCT image via a data link to the display device; thereafter, the display device renders the first A-scan signal of the OCT image in real time and displays a result. It would be appreciated that, as the legacy built-in processor, the FPGA has a faster signal processing speed.

In the embodiments, the digital signal processing performed by the onboard FPGA for the 3D OCT raw scanning data includes, but is not limited to: extracting phase information of the clock signal using Hilbert transform, and generating a time index corresponding to the clock signal based on the phase information of the equidistant clock signal, where the phase information of the clock signal is, for example, $0, \pi, 2\pi \ldots n\pi$, and the time index corresponding thereto is $t_1, t_2 \ldots t_n$; calculating respective values of the signal at $t_1, t_2 \ldots t_n$ using a difference algorithm, to obtain an interpolated signal, and performing dispersion compensation, FFT and logarithmic normalization operations on the interpolated signal, respectively, to obtain the first A-scan signal of the OCT image.

In the acquisition process of the 3D OCT raw scanning data, since the signal acquisition card collects a great amount of 3D OCT raw scanning data and directly transmits the collected 3D OCT raw scanning data to the display device, this can significantly increases the transmission cost while lowering the system reliability. In the embodiment, the signal processing is performed at the FPGA end, and the first A-scan signal of the OCT image obtained through the FPGA processing is sent to the display device for 3D OCT image display, which can reduce the data transmission rate, thus reducing the data transmission delay and OCT image display delay while improving the system reliability. In addition, in order to ensure the timeliness of the data, there is a high requirement imposed on the computing capability of the display device. In the embodiment, signal processing is performed at the FPGA end, thus lowering the data processing pressure on the display device and further reducing the requirement for the performance of the display device.

Steps S330: displaying a 3D OCT image based on the obtained first A-scan signal of the OCT image.

As an optional embodiment, after the first A-scan signal of the OCT image is obtained, the signal processor transmits the obtained first A-scan signal of the OCT image to the display device of the ophthalmic surgical microscope according to an SDI (serial digital interface) signal transmission protocol, to display a corresponding 3D OCT image.

In the embodiment of the present disclosure, after the 3D OCT raw scanning data is converted into the first A-scan signal, the first A-scan signal can be converted into an SDI format for transmission; then, the first A-scan signal in the SDI format is transmitted to the display device in a serial form; upon receiving the first A-scan signal in the SDI format, the display device decodes it and displays it in real time.

The ultra-high speed transmission protocol adopted in the legacy OCT transmission may involve a PCIE (peripheral component interconnect express), a Thunderbolt 4 port and the like, where the transmission distance of the PCIE is less than 50cm, and the transmission distance of the Thunderbolt 4 port is less than 2m. In contrast, the transmission distance of the SDI signal transmission protocol according to this embodiment is less than 300 m, and since the distance between the signal acquisition card and the display device in the surgical microscope is greater than the transmission range of the PCIE and the Thunderbolt 4 port, the transmission according to the SDI signal transmission protocol can meet the requirement of the system. In addition, the SDI signal transmission protocol supports a maximum transmission rate of 12Gbps, which can meet the requirement of the system for the transmission rate for 2D or 3D data.

By performing, by an external signal processor, signal processing on 3D OCT raw scanning data, the technical solution according to the embodiments of the present disclosure can reduce the data transmission cost, improve the system reliability, and reduce the requirement for the performance of the display device.

Fig. 8 is a schematic diagram of a structure of an intraoperative OCT navigation system for an ophthalmic surgical microscope provided by an embodiment of the present disclosure, where the intraoperative OCT navigation system for the ophthalmic surgical microscope according to the embodiment can perform the intraoperative OCT navigation method for the ophthalmic surgical microscope provided by the respective embodiments described above. As shown therein, the intraoperative OCT navigation system for the ophthalmic surgical microscope includes:

a scanning galvanometer 410 configured to obtain 3D OCT raw scanning data of a patient's ocular region;

a signal processing device 420 configured to perform signal processing on the obtained 3D OCT raw scanning data to obtain a first A-scan signal of an OCT image; and

a display device 430 configured to display a 3D OCT image based on the first A-scan signal of the OCT image.

In the technical solution according to the embodiment of the present disclosure, 3D OCT raw scanning data of a patient's ocular region is obtained using a scanning galvanometer, signal processing is performed on the obtained 3D OCT raw scanning data to obtain a first A-scan signal of an OCT image, and a 3D OCT image is displayed based on the first A-scan signal of the OCT image, thus enabling intraoperative 3D OCT navigation. As compared with 2D OCT navigation, the intraoperative 3D OCT navigation can improve the OCT display effect of the ophthalmic surgical microscope.

As an optional embodiment, the scanning galvanometer 410 can be specifically configured to: control the scanning galvanometer to perform 3D OCT scanning in a spiral scanning mode to obtain corresponding 3D OCT raw scanning data.

As an optional embodiment, the scanning galvanometer 410 can be specifically configured to:

control the scanning galvanometer to perform 3D OCT scanning on the patient's ocular region in a grid scanning mode to obtain the 3D OCT raw scanning data corresponding to the patient's ocular region.

As an optional embodiment, the scanning galvanometer 410 can be specifically configured to: in response to that the ophthalmic surgical microscope starts a 3D OCT scanning mode or is switched from a 2D OCT scanning mode to the 3D OCT scanning mode, obtain the 3D OCT raw scanning data corresponding to the patient's ocular region.

As an optional embodiment, the system further includes:

a 2D scanning data obtaining module configured, in response to that the ophthalmic surgical microscope starts the 2D OCT scanning mode or is switched from the 3D OCT scanning mode to the 2D OCT scanning mode, to obtain 2D OCT raw scanning data;

a 2D scanning data processing module configured to perform signal processing on the obtained 2D OCT raw scanning data to obtain a second A-scan signal of the OCT image; and

a 2D OCT image display module configured to display a 2D OCT image based on the second A-scan signal of the OCT image.

As an optional embodiment, the system further includes:

a 2D sweeping rate changing module configured, in the 3D OCT scanning mode, to change a sweeping rate of a variable-frequency light source of the ophthalmic surgical microscope to 400K; and/or

a 3D sweeping rate changing module configured, in the 2D OCT scanning mode, to change the sweeping rate of the variable-frequency light source of the ophthalmic surgical microscope to 100K.

As an optional embodiment, the signal processing device 420 may include an external signal processor,
such that signal processing can be performed by the external signal processor for the obtained 3D OCT raw scanning data to obtain the first A-scan signal of the OCT image.

Alternatively, the signal processing device 420 may include a built-in signal processor such that signal processing can be performed by the built-in signal processor for the obtained 3D OCT raw scanning data to obtain the first A-scan signal of the OCT image.

As an optional embodiment, the system further includes:
a signal transmission module configured to transmit the first A-scan signal of the OCT image obtained by the signal processor to a display device of the ophthalmic surgical microscope, according to an SDI signal transmission protocol, to display the corresponding 3D OCT image.

As an optional embodiment, the display device 430 is specifically configured to:
display the 3D OCT image in real time after converting the obtained first A-scan signal of the OCT image into corresponding grid point data.

The intraoperative OCT navigation system for the ophthalmic surgical microscope provided by the embodiments of the present disclosure can perform the intraoperative OCT navigation method for the ophthalmic surgical microscope provided by any of the embodiments of the present disclosure described above, which has functional modules and advantageous effects corresponding to the method.

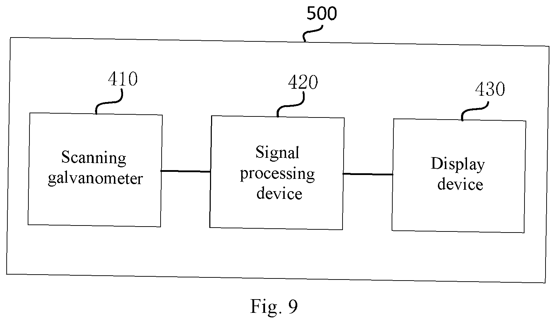

Fig. 9 is a schematic diagram of a structure of an ophthalmic surgical microscope provided by embodiments of the present disclosure. The ophthalmic surgical microscope 500 includes the intraoperative OCT navigation system for the ophthalmic surgical microscope provided by any of the embodiments described above, where the intraoperative OCT navigation system for the ophthalmic surgical microscope includes:

a scanning galvanometer 410 configured to obtain 3D OCT raw scanning data of a patient's ocular region;

a signal processing device 420 configured to perform signal processing on the obtained 3D OCT raw scanning data to obtain a first A-scan signal of an OCT image; and

a display device 430 configured to display a 3D OCT image based on the first A-scan signal of the OCT image.

In the technical solution according to the embodiment of the present disclosure, 3D OCT raw scanning data of a patient's ocular region is obtained using a scanning galvanometer, signal processing is performed on the obtained 3D OCT raw scanning data to obtain a first A-scan signal of an OCT image, and a 3D OCT image is displayed based on the first A-scan signal of the OCT image, thus enabling intraoperative 3D OCT navigation. As compared with 2D OCT navigation, the intraoperative 3D OCT navigation can improve the OCT display effect of the ophthalmic surgical microscope.

The specific implementations described above do not formulate any limitation to the scope of protection of the present disclosure. It would be appreciated by those skilled in the art that various modifications, combinations, sub-combinations and substitutions are allowed according to the design requirements and other factors. Any modification, equivalent replacement, improvement and the like made within the spirit ad principle of the present disclosure should all fall into the scope of protection of the present disclosure.

**Claims**

1. An intraoperative OCT navigation method for an ophthalmic surgical microscope, comprising:

obtaining, by a scanning galvanometer, 3D OCT raw scanning data of a patient's ocular region;
performing signal processing on the obtained 3D OCT raw scanning data to obtain a first A-scan signal of an OCT image; and
displaying a 3D OCT image based on the obtained first A-scan signal of the OCT image.

2. The method of claim 1, wherein obtaining, by the scanning galvanometer, the 3D OCT raw scanning data comprises:
controlling the scanning galvanometer to perform 3D OCT scanning in a spiral scanning mode to obtain corresponding 3D OCT raw scanning data.

3. The method of claim 1, wherein obtaining, by the scanning galvanometer, the 3D OCT raw scanning data of the patient's ocular region comprises:
controlling the scanning galvanometer to perform 3D OCT scanning on the patient's ocular region in a grid scanning mode to obtain the 3D OCT raw scanning data corresponding to the patient's ocular region.

4. The method of any of claims 1-3, wherein obtaining, by the scanning galvanometer, the 3D OCT raw scanning data of the patient's ocular region comprises:
in response to that the ophthalmic surgical microscope starts a 3D OCT scanning mode or is switched from a 2D OCT scanning mode to the 3D OCT scanning mode, obtaining, by the scanning galvanometer, the 3D OCT raw scanning data corresponding to the patient's ocular region.

5. The method of claim 4, further comprising:

in response to that the ophthalmic surgical microscope starts the 2D OCT scanning mode or is switched from the 3D OCT scanning mode to the 2D OCT scanning mode, obtaining, by the scanning galvanometer, 2D OCT raw scanning data;
performing signal processing on the obtained 2D OCT raw scanning data to obtain a second A-scan signal of an OCT image; and
displaying a 2D OCT image based on the second A-scan signal of the OCT image.

6. The method of claim 4, further comprising:

in the 3D OCT scanning mode, changing a sweeping rate of a frequency- variable light source of the ophthalmic surgical microscope to 400K; and/or
in the 2D OCT scanning mode, changing the sweeping rate of the frequency- variable light source of the ophthalmic surgical microscope to 100K.

7. The method of claim 1, wherein performing signal processing on the obtained 3D OCT raw scanning data to obtain the first A-scan signal of the OCT image comprises:
performing, by an external signal processor, signal processing on the obtained 3D OCT raw scanning data to obtain the first A-scan signal of the OCT image.

8. The method of claim 7, further comprising: after obtaining the first A-scan signal of the OCT image, transmitting the first A-scan signal of the OCT image obtained by the signal processor to a display device of the ophthalmic surgical microscope, according to an SDI signal transmission protocol, to display the corresponding 3D OCT image.

9. The method of claim 2, wherein displaying the 3D OCT image based on the first A-scan signal of the OCT image comprises:
displaying the 3D OCT image in real time after converting the obtained first A-scan signal of the OCT image into corresponding grid point data.

10. An intraoperative OCT navigation system for an ophthalmic surgical microscope, comprising:

a scanning galvanometer configured to obtain 3D OCT raw scanning data of a patient's ocular region;
a signal processing device configured to perform signal processing on the obtained 3D OCT raw scanning data to obtain a first A-scan signal of an OCT image; and
a display device configured to display a 3D OCT image based on the first A-scan signal of the OCT image.

11. An ophthalmic surgical microscope, comprising the intraoperative OCT navigation system for the ophthalmic surgical microscope of claim 10.

Obtain, by a scanning galvanometer, 3D OCT raw scanning data of a patient's ocular region — S110

Perform signal processing on the obtained 3D OCT raw scanning data to obtain a first A-scan signal of an OCT image — S120

Display a 3D OCT image based on the obtained first A-scan signal of the OCT image — S130

Fig. 1

Fig. 2A

Fig. 2B

Fig. 2C

Fig. 2D

Fig. 3

Fig. 4

```
┌─────────────────────────────────────────────────┐
│                                                 │
│  Control the scanning galvanometer to perform   │
│  3D OCT scanning in a spiral scanning mode to    │   ∼ S210
│  obtain corresponding 3D OCT raw scanning data   │
│                                                 │
└─────────────────────────────────────────────────┘
                         │
                         ▼
┌─────────────────────────────────────────────────┐
│                                                 │
│  Perform signal processing on the obtained 3D   │
│  OCT raw scanning data to obtain a first A-scan   │   ∼ S220
│  signal of an OCT image                          │
│                                                 │
└─────────────────────────────────────────────────┘
                         │
                         ▼
┌─────────────────────────────────────────────────┐
│                                                 │
│  Display a 3D OCT image based on the first       │
│  A-scan signal of the OCT image                  │   ∼ S230
│                                                 │
└─────────────────────────────────────────────────┘
```

Fig. 5

Fig. 6

Obtain, by a scanning galvanometer, 3D OCT raw scanning data of a patient's ocular region — S310

Perform, by an external signal processor, signal processing on the obtained 3D OCT raw scanning data to obtain a first A-scan signal of an OCT image; and — S320

Display a 3D OCT image based on the obtained first A-scan signal of the OCT image — S330

Fig. 7

410

Scanning galvanometer

420

Signal processing device

430

Display device

Fig. 8

500

410 420 430

| Scanning galvanometer | Signal processing device | Display device |

Fig. 9

## INTERNATIONAL SEARCH REPORT

| International application No. |
| --- |
| **PCT/CN2024/116613** |

### A. CLASSIFICATION OF SUBJECT MATTER

A61B3/10(2006.01)i; A61B3/13(2006.01)i; A61B34/20(2016.01)i; G01B9/02(2022.01)i

According to International Patent Classification (IPC) or to both national classification and IPC

### B. FIELDS SEARCHED

Minimum documentation searched (classification system followed by classification symbols)

IPC:A61B,A61F9,G01B9

Documentation searched other than minimum documentation to the extent that such documents are included in the fields searched

Electronic data base consulted during the international search (name of data base and, where practicable, search terms used)

CJFD, CNTXT, DWPI, ENTXT, ENTXTC, VEN, WPABS, WPABSC: 显微, 手术, 三维, 3维, 4维, 四维, 实时, 螺旋, 网格, A 扫, A? 扫, A SCAN+, A?SCAN+, 3D, 4D, OCT, spiral+, scan+, grid+

### C. DOCUMENTS CONSIDERED TO BE RELEVANT

| Category* | Citation of document, with indication, where appropriate, of the relevant passages | Relevant to claim No. |
| --- | --- | --- |
| PX | CN 117084623 A (TOWARDPI MEDICAL TECHNOLOGY LTD.) 21 November 2023 (2023-11-21) <br> claims 10-11 | 10-11 |
| X | WO 2021131020 A1 (NIKON CORP.) 01 July 2021 (2021-07-01) <br> description, paragraphs 14-95, and figures 1-17 | 10-11 |
| X | US 2017238798 A1 (NIDEK CO., LTD.) 24 August 2017 (2017-08-24) <br> description, paragraphs 23-148, and figures 1-11 | 10-11 |
| A | CN 110638527 A (SUZHOU INSTITUTE OF BIOMEDICAL ENGINEERING AND TECHNOLOGY, CHINESE ACADEMY OF SCIENCES) 03 January 2020 (2020-01-03) <br> entire document | 10-11 |
| A | US 2013271757 A1 (KANG JIN UNG et al.) 17 October 2013 (2013-10-17) <br> entire document | 10-11 |
| A | WO 2019112256 A1 (KOREA PHOTONICS TECHNOLOGY INSTITUTE) 13 June 2019 (2019-06-13) <br> entire document | 10-11 |

☐ Further documents are listed in the continuation of Box C.    ☑ See patent family annex.

| | |
| --- | --- |
| \*   Special categories of cited documents: <br> "A"   document defining the general state of the art which is not considered to be of particular relevance <br> "D"   document cited by the applicant in the international application <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
| --- | --- |
| **05 November 2024** | **15 November 2024** |

| Name and mailing address of the ISA/CN | Authorized officer |
| --- | --- |
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/116613**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **1-9**
because they relate to subject matter not required to be searched by this Authority, namely:

Claims 1-9 set forth an intraoperative OCT navigation method for use in an ophthalmic surgical microscope, which method is a method for treatment of the human or animal body by surgery, and relates to subject matter for which no search is required by the International Searching Authority as defined in PCT Rule 39.1.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2024/116613**

| Patent document cited in search report | | | Publication date (day/month/year) | Patent family member(s) | | | Publication date (day/month/year) |
|---|---|---|---|---|---|---|---|
| CN | 117084623 | A | 21 November 2023 | | None | | |
| WO | 2021131020 | A1 | 01 July 2021 | JP | 2024088714 | A | 02 July 2024 |
| | | | | JPWO | 2021131020 | A1 | 01 July 2021 |
| US | 2017238798 | A1 | 24 August 2017 | US | 10238281 | B2 | 26 March 2019 |
| CN | 110638527 | A | 03 January 2020 | EP | 3984486 | A1 | 20 April 2022 |
| | | | | EP | 3984486 | A4 | 24 August 2022 |
| | | | | JP | 2022539784 | A | 13 September 2022 |
| | | | | JP | 7350103 | B2 | 25 September 2023 |
| | | | | WO | 2021000466 | A1 | 07 January 2021 |
| | | | | US | 2022117696 | A1 | 21 April 2022 |
| US | 2013271757 | A1 | 17 October 2013 | WO | 2012088320 | A2 | 28 June 2012 |
| | | | | WO | 2012088320 | A3 | 13 December 2012 |
| WO | 2019112256 | A1 | 13 June 2019 | KR | 20190066922 | A | 14 June 2019 |
| | | | | KR | 102045880 | B1 | 18 November 2019 |

Form PCT/ISA/210 (patent family annex) (July 2022)